Europäisches Patentamt

European Patent Office

Office européen des brevets

(11) Veröffentlichungsnummer: **0 051 163**
A2

(12) # EUROPÄISCHE PATENTANMELDUNG

(21) Anmeldenummer: **81108102.5**

(22) Anmeldetag: **09.10.81**

(51) Int. Cl.³: **A 61 K 9/08**
**A 61 K 31/135**

(30) Priorität: **03.11.80 CH 8153/80**

(43) Veröffentlichungstag der Anmeldung:
**12.05.82 Patentblatt 82/19**

(84) Benannte Vertragsstaaten:
**AT BE CH DE FR GB IT LI NL SE**

(71) Anmelder: **Dr. Franz Köhler Chemie KG**
**Neue Bergstrasse 5 - 7**
**D-6146 Alsbach/Bergstrasse(DE)**

(72) Erfinder: **Köhler, Franz, Dr.**
**Neue Bergstrasse 5**
**D-6146 Alsbach-Bergstrasse(DE)**

(74) Vertreter: **Zutter, Hans Johann Niklaus**
**EPROVA AG Forschungsinstitut Im Laternenacker 5**
**CH-8200 Schaffhausen(CH)**

(54) **Intramuskulär schmerzlos anwendbare 4-Dimethylaminophenol Hydrochlorid-Lösung und Verfahren zu deren Herstellung.**

(57) Die Erfindung betrifft eine 4-Dimethylamino-phenol·Hydrochlorid-Lösung, die bei der intramuskulären Verabreichung keine Schmerzen verursacht und keine Gewebsnekrosen auslöst, dadurch charakterisiert, dass sie nur geringe Mengen von Wasser enthält oder wasserfrei ist und dass als Lösungsmittel physiologisch verträgliche niedere Alkanole, Glycerin, Glykole, Glykoläther, hydrophile Amide, Ester, Äther, Sulfone oder Sulfoxyde oder Fettsäureester oder ungesättigte Oele oder in der Regel Mischungen solcher Lösungsmittel verwendet werden.

Die Erfindung betrifft auch das Verfahren zur Herstellung solcher schmerzfrei injizierbarer Lösungen durch Auflösen von 4-Dimethylaminophenol·Hydrochlorid in den genannten Lösungsmitteln und der Weiterverarbeitung zu Injektionslösungen unter den gebotenen hygienischen Bedingungen.

EP 0 051 163 A2

Croydon Printing Company Ltd.

- 1 -

Intramuskulär schmerzlos anwendbare 4-Dimethylaminophenol·
Hydrochlorid-Lösung und Verfahren zu deren Herstellung.

Gegenstand der vorliegenden Erfindung ist eine intramuskulär schmerzlos anwendbare Lösung enthaltend 4-Dimethylaminophenol·Hydrochlorid sowie das Verfahren zur Herstellung derselben.

4-Dimethylaminophenol·Hydrochlorid (4-DMAP·HCl) hat eine
erhebliche Bedeutung als Entgiftungsmittel insbesondere
zur Behandlung von Blausäurevergiftungen erlangt.

Bei der Einwirkung von Blausäure, Cyaniden und Nitrilen
auf den Organismus kommt es durch Blockierung des ferrihaltigen Atmungsfermentes Cytochromoxidase zu einer sehr
rasch einsetzenden Hemmung des oxidativen Stoffwechsels.
Dieser Prozess führt zu einem vollständigen Stillstand
der physiologischen Funktion der Zelle, besonders im Bereiche des Zentralnervensystems und des Myokards. Das
Hämoglobin verliert die Fähigkeit, den Sauerstoff frei zu
geben, weshalb es nach anfänglich gegenregulatorischen
Hyperventilation zu Krämpfen, Opisthotonus, Atem- und
Herzstillstand kommt.

Die Inhibierung der Cytochromoxidase durch das Cyanid-Ion
oder -Radikal ist reversibel: der Vorgang unterliegt einem Gleichgewicht, das durch Affinitätsunterschiede des Cyanid-Ions zum dreiwertigen Eisen der Cytochromoxidase auf der einen und zum Ferrihämoglobin (Methämoglobin) auf der anderen Seite charakterisiert ist. Dabei wird Cyanid von der Cytochromoxidase weniger fest gebunden, als vom Ferrihämoglobin. Dieser quantitative Unterschied in der Bindungsfestigkeit war der entscheidende Wegweiser für die Entwicklung geeigneter Antidota zur Therapie von Vergiftungen mit Blausäure, ihren Salzen und organischen Derivaten.

In eingehenden Untersuchungen haben

N. Weger: Cyanidvergiftung und Therapie. Wehrmed. Monatsschrift, 19 (1975), 6;

M. Daunderer, H. Theml u. N. Weger: Behandlung der Blausäurevergiftung durch 4-Dimethylaminophenol (4-DMAP). Med.Klin.,·69 (1974), 1626;

D. Christel, P. Eyer, M. Hegemann, M. Kiese, W. Lörcher u. N. Weger: Pharmacokinetics of Cyanide in Poisoning of Dogs, and the Effect of 4-Dimethylaminophenol or Thiosulfate. Arch. Toxicol., 38 (1977), 177;

N. Weger: Antidote bei akuten Vergiftungen. Fortschr. Med., 95 (1977), 2407;

N. Weger: Therapie der Blausäurevergiftung. Med. Monatsschrift 23 (1969), 436.

gezeigt, dass 4-Dimethylaminophenol·Hydrochlorid das zur Zeit am zuverlässigsten und am besten wirksame Antidot bei einschlägigen Vergiftungen darstellt.

Da im Falle einer akzidentiellen oder suizidalen Vergiftung mit Blausäure, Cyaniden oder Nitrilen nur die parenterale Applikation einer injizierbaren Zubereitung von 4-Dimethylaminophenol·Hydrochlorid praktizierbar ist, muss erwartet werden, dass diese Zubereitung sowohl intravenös als auch intramuskulär ohne klinische Komplikationen appliziert werden kann. Dabei hat die intramuskuläre Gabe einen besonders hohen Stellenwert, weil in den meisten Fällen ein fachgerechte intravenöse Behandlung lege artis, wegen Abwesenheit eines Arztes oder eines mit der intravenösen Technik ausreichend Bewanderten, nicht möglich ist. Demgegenüber kann die intramuskuläre Applikation im Notfall auch von einem Laien ausgeführt werden. Dieser Sachverhalt ist besonders bei Massenvergiftungen, etwa bei kriegerischen Auseinandersetzungen, von ganz entscheidender Bedeutung.

Während die intravenöse Zufuhr einer wässrigen Lösung von 4-Dimethylaminophenol·Hydrochlorid in geeigneter Konzentration keine lokalen Schmerzen oder andere unerwünschte Sensationen verursacht und innerhalb weniger Minuten den therapeutisch notwendigen Wirkspiegel von 30 - 50 % Ferrihämoglobin aufbaut, ist die intramuskuläre Applikation einer derartigen handelsüblichen Lösung von 250 mg 4-Dimethylaminophenol·Hydrochlorid in 5 ml Aqua iniectabilia mit einem ausserordentlich intensiven, stundenlang anhaltenden lokalen Schmerz verbunden, sowie mit örtlichem Gewebstod im Bezirk des Applikationsortes, der als körnige Nekrose in Erscheinung tritt. Das bedeutet, dass eine 4-Dimethylaminophenol·Hydrochlorid-Lösung zur Verfügung stehen muss, die bei der intramuskulären Applikation, gegebenenfalls mit einem leicht anwendbaren Autoinjektor, frei von lokal auftretenden Schmerzen ist, aus der der Wirkstoff rasch resorbiert wird, um innerhalb weniger Minuten die erforderliche maximale Ferrihämoglobinkonzentration von 30 - 50 % zu erreichen, die keine Gewebsnekrosen verursacht, die eine ausreichende Stabilität, das heisst, eine lange Haltbarkeit aufweist, die nicht nur intramuskulär, sondern auch intravenös applizierbar ist und die zur schmerzfreien intramuskulären Anwendung ein Lokalanästhetikum nicht benötigt.

Es ist davon auszugehen, dass die intensive, lang anhaltende Schmerzreaktion und die markante Nekrotisierung des Gewebes am Injektionsort, durch den hohen Dissoziationsgrad im sauren Bereich der handelsüblichen 5 proz. wässrigen 4-Dimethylaminophenol·Hydrochlorid-Lösung verursacht wird. Zwar lässt sich durch Anheben des pH-Wertes die lokale Verträglichkeit geringfügig erhöhen, doch wird bei einer derartigen Massnahme die Stabilität der reinwässrigen 4-Dimethylaminophenol·Hydrochlorid-Lösung erheblich herabgesetzt, was zur Folge hat, dass derartige Lösungen nur in Stickstoffatmosphäre und unter Mitverwendung von Antioxidantien galenisch verarbeitbar sind. Die klinisch gestellten Anforderungen werden dabei nicht erreicht.

Es wurde gefunden, dass sich eine unter den üblichen Lagerungsbedingungen stabile, völlig schmerzfrei intramuskulär injizierbare, keine Gewebsnekrose verursachende 4-Dimethylaminophenol·Hydrochlorid Lösung herstellen lässt, wenn deren Wasseranteil drastisch auf eine geringe Menge reduziert oder ganz weggelassen wird. Dadurch wird die Protonierung des Phenols, die Dissoziation, weitgehend oder völlig ausgeschaltet und dessen lokale Gewebsindifferenz optimal zur Geltung gebracht.

Als Lösungsmittel zur Herstellung von schmerzfrei und gewebsinert intramuskulär injizierbaren 4-Dimethylaminophenol·Hydrochlorid-Lösungen eignen sich wässrig-organisch sowie wasserfreie Lösungsmittel, soweit diese aus pharmakologischen, toxikologischen, pharmakodynamischen und pharmakokinetischen Gesichtspunkten in Betracht kommen.
Voraussetzungen sind einmal ihre Lösungsfähigkeit für 4-Dimethylaminophenol·Hydrochlorid, ihre minimale Toxizität, optimale Gewebsverträglichkeit sowie im allgemei-

- 5 -

nen die Mischbarkeit mit Wasser bzw. mit Körperflüssigkeit insbesondere mit Serum.

Bei der Bemessung des Lösungsmittelanteiles sind bestimmte Kautelen zu beachten, damit der erfindungsbegründende
Effekt der Schmerzlosigkeit bei der intramuskulären Verabreichung der Lösungen erreicht wird: der Wasseranteil
ist so gering als möglich zu halten. Er soll 30 Volumprozente (Vol%) nicht überschreiten, der Äthanolanteil
soll, zur Vermeidung von Gewebsreizungen 30 Vol% nicht
überschreiten.

Optimalerweise soll der Wassergehalt auf 10 Vol% begrenzt
oder vorzugsweise ganz weggelassen werden.
Den Hauptanteil der Lösungsmittel bilden gemäss vorliegender Erfindung Glykole und Glykoläther.

Unter den vorliegenden Voraussetzungen sind folgende, für
die Herstellung von Injektionslösungen bereits bewährte
und von der Fachwelt akzeptierte, Lösungsmittel geeignet
(vergleiche dazu: J. Anschel, Pharm. Ind. 1965, 27(11a),
781-7 [Chemical Abstracts 67 (1967) 120154h]):
a) niedrige Alkanole, und zwar Äthanol und Isopropanol,
b) Glycerin, Glykole insbesondere 1,2-Propandiol, 1,3-
   Butendiol, Tetraäthylenglykol, Äthylenglykol, Triäthylenglykol, Polyäthylenglykol, Diäthylenglykol,
   Hexenylglykol, Isopropylenglykoläther, Diäthylenglykoldimethyläther (Diglyme),
c) Benzylalkohol,
d) niedrige hydrophile Amide wie N,N-Dimethylacetamid
   (DMAC), N,N-Dimethylformamid (DMF), N,N-Dimethylmethoxyacetamid, N-(β-Hydroxyäthyl)-laktamid,
e) hydrophile Ester, Äther, Sulfone und Sulfoxyde wie
   Äthyllaktat, Äthylacetat, Tetrahydrofurfurylmethyl-

151/CH

äther, 2,2-Dimethyl-4-hydroxymethyl-1,3-Dioxolan
(= Isopropylidenglycerin), Tetrahydrothiophen-1,1-
dioxyd und etwa Dimethylsulfoxyd (DMSO).

Die mit diesen Lösungsmitteln bereiteten Lösungen sind sowohl für die intramuskuläre als auch die intravenöse Verabreichung geeignet und demnach in Notfällen sowohl durch den Kameraden oder Sanitäter als auch den Arzt verwendbar.

Es wurde gefunden, dass sich auch ölige Lösungen von 4-Dimethylaminophenol·Hydrochlorid herstellen lassen, wenn man als Lösungsmittel Mischungen von natürlichen oder synthetischen Oelen mit N,N-Dimethylacetamid, N,N-Dimethylformamid, 2,2-Dimethyl-4-hydroxymethyl-1,3-dioxolan, Tetrahydrothiophen-1,1-dioxyd, Dimethylsulfoxyd oder Äthyllaktat benutzt. Es ist einleuchtend, dass diese Lösungen nur für die intramuskuläre Verabreichung geeignet sind.

Gegenstand der Erfindung ist demnach eine intramuskulär schmerzlos anwendbare 4-Dimethylaminophenol·Hydrochlorid-Lösung, dadurch gekennzeichnet, dass sie nur geringe Mengen von Wasser enthält oder wasserfrei ist und als Lösungsmittel physiologisch verträgliche niedere Alkanole, Glycerin, Glykole, Glykoläther, Benzylalkohol, niedere hydrophile Amide, Ester, Äther, Sulfone und/oder Sulfoxyde oder Fettsäureester oder ungesättigte Oele oder Mischungen solcher Lösungsmittel enthält.
Der Wassergehalt der erfindungsgemässen 4-DMAP·HCl-Lösung beträgt 0 (Null) bis 30 Vol%.
Die Lösung kann als Alkanol, Äthanol oder Isopropanol, als Glykol, 1,2-Propandiol oder 1,3-Butandiol, als Glykoläther, Tetraäthylenglykol, Äthylenglykol, Triäthylenglykol, Polyäthylenglykol, Isopropylenglykoläther oder Diäthylenglykoldimethyläther oder Glycerin enthalten.

Als niedrige Amide, Ester, Äther, Sulfone oder Sulfoxyde kann die DMAP·HCl-Lösung N,N-Dimethylacetamid, N,N-Dimethylformamid, N-(β-Hydroxyäthyl)-laktamid, N,N-Dimethylmethoxyacetamid, Äthyllaktat, Tetrahydrofurfurylmethyläther, 2,2-Dimethyl-4-hydroxymethyl-1,3-dioxolan, Tetrahydrothiophen-1,1-dioxyd und/oder Dimethylsulfoxyd oder Mischungen davon enthalten.

Das Verfahren zur Herstellung einer intramuskulär schmerzlos anwendbaren 4-Dimethylaminophenol·Hydrochlorid-Lösung ist dadurch gekennzeichnet, dass man 4-Dimethylaminophenol·Hydrochlorid in den genannten Lösungsmitteln bzw. Mischungen derselben löst und nach an und für sich bekannten Methoden zur sterilen intramuskulär und in der Regel auch intravenös verabreichbaren Injektionslösung verarbeitet.

Die Verwendung der meisten oben genannten Lösungsmittel zur Herstellung von Lösungen pharmazeutischer Wirkstoffe ist bereits in der Literatur beschrieben worden, jedoch stets in einem Zusammenhang der nichts zu tun hat mit der Zielsetzung der vorliegenden Erfindung der Herstellung von schmerzlos intramuskulär applizierbaren 4-DMAP·HCl-Lösungen.

G. Bormann et al, Arzneimittel-Forschung 8 (1958), 276-277 benutzten Glykole als Lösungsmittel für das in Wasser schwer lösliche Meprobamat (2-Methyl-2-propyl-1,3-propandioldicarbonat).

H. Spiegelberg et al, Arzneimittel-Forschung 6 (1956), 75-77 haben Glucofural, das sind Polyäthylenglykoläther des Tetrahydrofurfurylalkohols benutzt zur Herstellung von stabilen Lösung für die Kombination 3-Pyridinmethanol/Acetylcholin. In wässriger Lösung ist Acetylcholin nicht haltbar.

W.A. Ritschel, Pharm. Ind. 1973, 35 (5 & 6), 273-85 und 363-9 [Chemical Abstracts 79 (1973) 83414t und 80 (1974) 19410g] hat eine Uebersicht über rasch und verzögert wirkende Injektionslösungen gegeben.

Zur Verdeutlichung der erfindungsgemäss beschriebenen 4-Dimethylaminophenol·Hydrochlorid-Lösung, deren intramuskuläre Anwendung, im Gegensatz zu der handelsüblichen, reinwässrigen 5 proz. Lösung, völlig schmerzfrei und ohne Bildung von Gewebsnekrosen, realisierbar ist, werden folgende Beispiele aufgeführt:

Beispiel 1: eine Lösung bestehend aus 12.5 g  4-Dimethylaminophenol·Hydrochlorid
      1.5 ml  Benzylalkohol
      30.0 ml  Aqua iniectabilia
  ad 100.0 ml  1,2-Propandiol.

Beispiel 2: eine Lösung bestehend aus 12.5 g  4-Dimethylaminophenol·Hydrochlorid
      4.0 ml  Benzylalkohol
      5.0 ml  Aqua iniectabilia
      30.0 ml  Polyäthylenglykol (200-600)
  ad 100.0 ml  1,3-Butandiol.

Beispiel 3: eine Lösung bestehend aus 12.5 g  4-Dimethylaminophenol·Hydrochlorid
      1.5 ml  Benzylalkohol
      30.0 ml  Äthylalkohol, absol.
  ad 100.0 ml  1,2-Propandiol.

Beispiel 4: eine Lösung bestehend aus 5.0 g  4-Dimethylaminophenol·Hydrochlorid
      10.0 ml  Äthylalkohol, absol.
  ad 100.0 ml  1,3-Butandiol.

151/CH

Beispiel 5:   eine Lösung bestehend aus 10.0 g   4-Dimethylaminophenol·Hydrochlorid 0.5 ml  Benzylalkohol 5.0 ml  Äthylalkohol, absol.

           2.0 ml  Aqua iniectabilia 50.0 ml  1,2-Propandiol ad 100.0 ml  1,3-Butandiol.


Beispiel 6:   eine Lösung bestehend aus 10.0 g   4-Dimethylaminophenol·Hydrochlorid 10.0 ml  Äthylalkohol, absol.

           8.0 ml  Polyäthylenglykol (200-600) 2.0 ml  Glycerin, 50 proz.

     ad 100.0 ml  1,3-Propandiol.


Beispiel 7:   eine Lösung bestehend aus 10.0 g   4-Dimethylaminophenol·Hydrochlorid

           6.0 ml  Äthylalkohol, absol.

          20.0 ml  Triäthylenglykol

          15.0 ml  Äthyllaktat

     ad 100.0 ml  1,2-Propandiol.


Beispiel 8:   eine Lösung bestehend aus 20.0 g   4-Dimethylaminophenol·Hydrochlorid 5.0 ml  Aqua iniectabilia 15.0 ml  Isopropylenglykoläther 10.0 ml  Tetrahydrofurfurylmethyläther 60.0 ml  1,3-Butandiol ad 100.0 ml  1,2-Propandiol.

Beispiel 9:   eine Lösung bestehend aus 12.5 g   4-Dimethylaminophenol·Hydrochlorid 4.0 ml   Benzylalkohol 0.05 g   Natriumbisulfit 10.0 ml   Aqua iniectabilia ad 100.0 ml   1,2-Propandiol.


Beispiel 10:   eine Lösung bestehend aus 12.5 g   4-Dimethylaminophenol·Hydrochlorid 4.0 ml   Benzylalkohol 0.05 g   Natriumbisulfit 30.0 ml   Aqua iniectabilia ad 100.0 ml   1,2-Propandiol.


Die vorstehend beschriebenen Lösungen von 4-DMAP·HCl sind sowohl für die intramuskuläre als auch die intravenöse Verabreichung geeignet.

Ausschliesslich für die intramuskuläre Applikation geeignet sind folgende 4-DMAP·HCl-Lösungen:

Beispiel 11:   eine Lösung bestehend aus 10.0 g   4-Dimethylaminophenol·Hydrochlorid

40.0 ml   Äthyllaktat

ad 100.0 ml   Rizinusöl.


Beispiel 12:   eine Lösung bestehend aus 10.0 g   4-Dimethylaminophenol·Hydrochlorid 60.0 ml   N,N-Dimethylacetamid ad 100.0 ml   Isopropylmyristinat.


Die Herstellung der intramuskulär schmerzlos applizierbaren und in der Regel auch intravenös verabreichbaren Lösungen

151/EPA

von 4-Dimethylaminophenol·Hydrochlorid (4-DMAP·HCl) erfolgt durch Auflösen dieses Wirkstoffes in den in den Beispielen angegebenen Arten und Mengen von Lösungsmitteln, dem Verdünnen der homogenen klaren Lösung mit dem am Schluss genannten Lösungsmittel (ad 100...), dem Filtrieren der erhaltenen Lösungen zur Abtrennung etwa von staubförmigen Verunreinigungen, dem Abfüllen in Ampullen, Serumfläschchen oder speziellen Behältern für die Autoinjektion und der Sterilisation der Lösungen. Alle Operationen müssen unter strengen hygienischen Bedingungen ausgeführt werden.

Patentansprüche

1. Intramuskulär schmerzlos anwendbare 4-Dimethylaminophenol·Hydrochlorid-Lösung, dadurch gekennzeichnet, dass sie nur geringe Mengen von Wasser enthält oder wasserfrei ist und als Lösungsmittel physiologisch verträgliche niedere Alkanole, Glycerin, Glykole, Glykoläther, Benzylalkohol, niedere hydrophile Amide, Ester, Äther, Sulfone und/oder Sulfoxyde oder Fettsäureester oder ungesättigte Oele oder Mischungen solcher Lösungsmittel enthält.

2. 4-Dimethylaminophenol·Hydrochlorid-Lösung zur schmerzlosen intramuskulären Injektion, gemäss Patentanspruch 1, dadurch gekennzeichnet, dass der Wassergehalt O (Null) bis 30 % beträgt.

3. 4-Dimethylaminophenol·Hydrochlorid-Lösung gemäss Patentanspruch 1, dadurch gekennzeichnet, dass sie als Alkanol Äthylalkohol oder Isopropanol enthält.

4. 4-Dimethylaminophenol·Hydrochlorid-Lösung gemäss Patentanspruch 1, dadurch gekennzeichnet, dass sie als Lösungsmittel im wesentlichen ein Glykol oder Glykole, Glykoläther, Glycerin oder Mischungen solcher Lösungsmittel enthält.

5. 4-Dimethylaminophenol·Hydrochlorid-Lösung gemäss Patentanspruch 1 und 4, dadurch gekennzeichnet, dass sie als Glykol bzw. Glykoläther 1,2-Propandiol, 1,3-Butandiol, Tetraäthylenglykol, Äthylenglykol, Tri-äthylenglykol, Polyäthylenglykol, Diäthylenglykol, Hexenylglykol, Isopropylenglykoläther und/oder Di-äthylenglykol-dimethyläther enthält.

151/EPA

6. 4-Dimethylaminophenol· Hydrochlorid-Lösung, gemäss Patentanspruch 1, dadurch gekennzeichnet, dass sie als niedere hydrophile Amide, Ester, Äther, Sulfone oder Sulfoxyde N,N-Dimethylacetamid, N,N-Dimethylformamid N-(β-Hydroxyäthyl)-laktamid, N,N-Dimethylmethoxyacetamid, Äthyllaktat, Äthylacetat, Tetrahydrofurfurylmethyläther, 2,2-Dimethyl-4-hydroxymethyl-1,3-dioxolan, Tetrahydrotriophen-1,1-dioxyd und/oder Dimethylsulfoxyd oder Mischungen davon enthält.

7. Verfahren zur Herstellung einer intramuskulär schmerzlos anwendbaren 4-Dimethylaminophenol·Hydrochlorid-Lösung gemäss Patentansprüchen 1 bis 6, dadurch gekennzeichnet, dass man 4-Dimethylaminophenol·Hydrochlorid in den genannten Lösungsmitteln bzw. Mischungen derselben löst und nach an und für sich bekannten Methoden zu sterilen intramuskulär und in der Regel auch intravenös verabreichbaren Injektionslösungen verarbeitet.